# EUROPÄISCHE PATENTSCHRIFT

(11) **EP 2 039 309 B1**
(45) Veröffentlichungstag und Bekanntmachung des Hinweises auf die Patenterteilung: **30.03.2011**
(21) Anmeldenummer: 08012924.0
(22) Anmeldetag: 17.07.2008
(51) Int. Cl.: A61B 17/50, A45C 11/24

(54) **Vorrichtung zum Entfernen von Parasiten, insbesondere von Zecken**
Device for removing parasites, in particular ticks
Dispositif d'extraction de parasites, en particulier de tiques

(30) Priorität: 20.09.2007 DE 102007045105
(43) Veröffentlichungstag der Anmeldung: 25.03.2009
(73) Patentinhaber: Dr. Schick Gmbh, 74889 Sinsheim (DE)
(72) Erfinder: Schick, Gerhard, 74889 Sinsheim (DE)
(74) Vertreter: Ullrich & Naumann

(56) Entgegenhaltungen:
- WO-A-03/022094
- WO-A-2004/054457
- WO-A-2007/129095
- US-A- 5 447 511

## Beschreibung

Die Erfindung betrifft eine Vorrichtung zum Entfernen von Parasiten, insbesondere von Zecken, vom Menschen oder vom Tier, mit einer zur Handhabung dienenden Karte und mit mindestens einem in einem Eckbereich der Karte ausgebildeten Aufnahmeschlitz zum Hintergreifen oder Ergreifen des Parasiten, insbesondere der Zecke bzw. des Zeckenkörpers.

Vorrichtungen der in Rede stehenden Art sind seit langem aus der Praxis bekannt und existieren in den unterschiedlichsten Formen und Größen. Dabei sind die Vorrichtungen häufig in Form von Pinzetten ausgebildet. Derartige Vorrichtungen finden ihre Verwendung sowohl im privaten als auch im gewerblichen Bereich immer dann, wenn Zecken von der menschlichen oder tierischen Haut während des Blutsaugens der Zecken zu entfernen sind. Eine solche Entfernung von Zecken von der Haut ist erforderlich, da durch den Stich der Zecken gefährliche Krankheiten auf Mensch und Haustiere übertragen werden können. Sowohl beim Entfernen mittels Finger als auch mittels Pinzette lässt sich meist nur der beim Saugakt stark anschwellende Zeckenkörper abreißen, während die Mundwerkzeuge der Zecke in der Haut stecken bleiben. Die Übertragung von Krankheiten ist in diesem Zustand möglich.

Eine vollständige Entfernung einer Zecke während des Blutsaugens lässt sich meist nur durch ein gefühlvolles Greifen bzw. Festhalten oder Hintergreifen und anschlieβendes Herausdrehen oder Herausziehen der Mundwerkzeuge der Zecke aus der Haut erreichen. Eine vorsichtige Handhabung ist auf jeden Fall erforderlich, um nämlich ein Abreißen der Mundwerkzeuge vom Zeckenkörper zu vermeiden.

Aus der EP 1 082 942 B1 ist eine Vorrichtung zum Entfernen vom Zecken bekannt, wobei es sich dabei im Konkreten um eine Zeckenzange mit einer entsprechenden Greifeinrichtung handelt. Zusätzlich ist dort auf der dem Greifbereich gegenüberliegenden Seite eine Aufnahmeeinrichtung vorgesehen, die mit einem Aufnahmeschlitz zum Hintergreifen des Zeckenkörpers ausgestattet ist. Die aus der EP 1 082 942 B1 bekannte Zeckenzange weist eine komplizierte Konstruktion auf und ist aufgrund ihrer Größe zum Einstecken, beispielsweise in die Hosentasche, wenig geeignet. Außerdem erschwert die beidseitige Ausgestaltung von Werkzeugen die Handhabung.

Aus der DE 297 22 310 U1 ist ein Zecken-Ziehgerät bekannt, welches aus einem formstabilen steifen Flachmaterial, beispielsweise aus Metall oder Kunststoff, besteht. Der zur Handhabung dienende Körper ist im Sinne eines Blättchens mit verjüngtem Fortsatz ausgeführt, der am freien Ende einen Aufnahmeschlitz zum Hintergreifen des Zeckenkörpers aufweist. Der Fortsatz ist gegenüber dem Hauptkörper gebogen, so dass die Vorrichtung für eine Art Hebelbewegung geeignet ist. Bei dem aus der DE 297 22 310 U1 bekannten Zecken-Ziehgerät ist die filigrane Ausgestaltung, insbesondere des sehr dünn ausgeführten Fortsatzes problematisch, da er bei unsachgemäßer Handhabung leicht verbiegt oder gar abbricht. Außerdem ist das Ziehgerät aufgrund des abgebogenen Fortsatzes nur bedingt zum Einstecken in die Hosentasche geeignet.

Eine gattungsbildende Vorrichtung ist aus der DE 202 21 252 U1 bekannt. Im Konkreten besteht die Vorrichtung im Wesentlichen aus einer viereckigen Karte, die die Größe und Form einer Scheckkarte hat. In mindestens einem Eckbereich der Karte ist ein Aufnahmeschlitz zum Hintergreifen der Zecke bzw. des Zeckenkörpers vorgesehen.

Bei dem in Fig. 6 der DE 202 21 252 U1 gezeigten Ausführungsbeispiel sind insgesamt zwei Aufnahmeschlitze vorgesehen. Der eine Aufnahmeschlitz ist direkt in einer Ecke der Karte ausgebildet. Ganz offensichtlich ist erkannt worden, dass die Vorkehrung dieses Aufnahmeschlitzes im Eckbereich der Karte nicht ausreicht, insbesondere dann, wenn sich die Zecke in einem nur schwer zugänglichen Bereich des Körpers eingenistet hat. So ist dort ein zweiter Aufnahmeschlitz vorgesehen, nämlich in einem benachbarten Eckbereich, wobei die Karte dort derart reduziert bzw. ausgenommen ist, dass der zweite Aufnahmeschlitz, ähnlich wie bei der DE 297 22 310 U1, am Ende eines schlanken Fortsatzes ausgebildet ist.

Die aus der DE 202 21 252 U1 bekannte Vorrichtung ist in der Praxis problematisch. Der in der Ecke rechtwinklig zueinander ausgebildeter Ränder vorgesehene Aufnahmeschlitz lässt sich aufgrund der durch die rechtwinklige Geometrie vorgegebenen Breite der Karte nur bedingt einsetzen. Der im schlankeren Bereich, d.h. in dem fingerartigen Fortsatz in der anderen Ecke ausgebildete Aufnahmeschlitz ist in einem stark reduzierten Materialbereich ausgebildet. Folglich besteht hier die Gefahr, dass dieser Bereich durch die Anwendung verbiegt oder abbricht. Handelt es sich bei dem Material der Karte um einen relativ weichen Kunststoff, so reicht die Festigkeit des den Aufnahmeschlitz tragenden Fortsatzes nicht aus, um die Zecke wirkungsvoll aus der Haut herausziehen zu können.

Aus der US-A-5,447,511 ist ebenfalls eine Vorrichtung zum Entfernen von Zecken bekannt, wobei diese Vorrichtung als langgestreckte Karte ausgeführt ist, die am freien Ende spitz zuläuft. Dort ist ein Aufnahmeschlitz für das Hinter- bzw. Untergreifen der Zecke vorgesehen.

Im Lichte der voranstehenden Ausführungen liegt der vorliegenden Erfindung die Aufgabe zugrunde, eine gattungsbildende Vorrichtung zum Entfernen von Parasiten, insbesondere von Zecken, derart auszugestalten und weiterzubilden, dass sich bei einfachster und dabei stabiler Konstruktion die Parasiten sicher entfernen lassen, ohne dass dabei die Funktionsfähigkeit der Vorrichtung bzw. Karte auch bei häufigem Gebrauch beeinträchtigt ist.

Erfindungsgemäß wird die voranstehende Aufgabe durch die Merkmale des Patentanspruchs 1 gelöst. Danach ist die in Rede stehende Vorrichtung dadurch gekennzeichnet, dass die Karte in Form eines gleichseitigen Dreiecks ausgeführt und der Aufnahmeschlitz in einer der Ecken ausgebildet ist.

Erfindungsgemäß ist erkannt worden, dass sich die im zuvor erörterten Stand der Technik auftretenden Probleme durch eine verblüffend einfache Maßnahme beheben lassen, nämlich dadurch, dass man die zur Handhabung dienende Karte dreieckig ausführt. Dies hat gegenüber der rechtwinkeligen Ausführung einer Karte, beispielsweise einer Karte im Scheck-/Kreditkartenformat, den enormen Vorteil, dass die Eckbereiche der Karte durch gleichlange Kanten gebildet sind, die einen Winkel von weniger als 90 Grad einschließen. Hier lässt sich der Aufnahmeschlitz ausbilden, so dass bei äußerst stabiler Ausführung - per se - eine wesentlich schlankere Ausgestaltung des Bereichs um den Aufnahmeschlitz herum möglich ist, ohne dazu einen filigranen Fortsatz in die Karte hineinzuarbeiten. So ist in erfindungsgemäßer Weise der Aufnahmeschlitz in einer der Ecken der Karte ausgebildet, so dass sich diese in idealer Weise zum Hintergreifen des Parasiten bzw. der Zecke eignet, wobei ein Herausziehen bzw. Heraushebein der Zecke aufgrund der hinreichenden Stabilität problemlos möglich ist. Aufgrund der Tatsache, dass die Karte als gleichseitiges Dreieck ausgeführt ist, ist einerseits die Ausbildung des Aufnahmeschlitzes in einem Bereich möglich, der relativ schlank ausgestaltet ist. Andererseits führt die gleichseitige Ausgestaltung des Dreiecks zu identischen Winkeln in den Ecken, so dass die Karte in den Eckbereichen, hinreichend stabil ist und es daher nicht erforderlich ist, den Schlitz in einen langgestreckten filigranen Bereich hinein zu arbeiten, in dem sich die Karte verbiegt oder gar abbricht. Aufgrund der gleichseitigen Ausgestaltung der dreieckigen Karte ist in den Eckbereichen eine hinreichende Stabilität realisiert.

Je nach verwendetem Material, sollte die Karte eine Dicke im Bereich von 1 bis 2 mm, vorzugsweise eine Dicke von 1,5 mm aufweisen, um nämlich eine einfache Handhabung sowie ein leichtes Verstauen der Karte, beispielsweise in einer Brieftasche oder einer Geldbörse, zu ermöglichen. Auf das zu verwendende Material wird später noch eingegangen werden.

In Bezug auf die Ausgestaltung des Aufnahmeschlitzes ist es von besonderem Vorteil, wenn sich dieser von einer breiten Öffnung bis zu einem spitz oder leicht abgerundet zulaufenden Ende vorzugsweise konisch oder bogenförmig (konkav oder konvex) verjüngt. Im Rahmen einer solchen Ausgestaltung ist gewährleistet, dass man die Karte mit dem Aufnahmeschlitz unter den Zeckenkörper schieben kann, um nämlich diesen zu hintergreifen. Da die Zecken insbesondere im vollgesogenen Zustand meist eng am Körper anliegen, ist es von weiterem Vorteil, wenn die den Aufnahmeschlitz bildenden Kanten vorzugsweise von einer Seite der Karte her abgeschrägt bzw. angefast sind. Dabei lässt sich die Karte eng an der Haut anliegend unter die Zecke schieben, wobei die Kanten des Aufnahmeschlitzes rampenartig wirken.

Insbesondere unter dem Gesichtspunkt einer stabilen Ausgestaltung, ist es von Vorteil, wenn der Aufnahmeschlitz im Sinne einer Winkelhalbierenden in die jeweilige Ecke der Karte eingearbeitet ist. Dies bedeutet mit anderen Worten, dass der Aufnahmeschlitz symmetrisch in der Ecke ausgebildet ist.

Insbesondere beim Herausziehen von Zecken ist zu beachten, dass gewährleistet sein muss, dass diese im unterschiedlichen Stadium wirksam herausziehbar, sind. Dabei ist die Erkenntnis von Bedeutung, dass sich die Larven, Nymphen und adulten Zecken nur äußerst schwierig mit dem gleichen Werkzeug aus dem Körper herausziehen lassen. Somit ist es von ganz besonderem Vorteil, wenn die Karte an zwei oder drei ihrer Ecken, vorzugsweise an allen Ecken, Aufnahmeschlitze in unterschiedlichen Größen, d.h. mit unterschiedlichen Öffnungen und Tiefen, aufweist. So lassen sich an der dreieckigen Karte drei unterschiedlich große Aufnahmeschlitze vorsehen, die den unterschiedlichen Größen der Zecke Rechnung tragen. Durch diese Maßnahme ist eine besonders sichere Handhabung in Bezug auf das Herausziehen der Zecke gewährleistet.

Wie bereits eingangs erwähnt, dient die erfindungsgemäße Vorrichtung zum grundsätzlichen Entfernen von Parasiten, hauptsächlich von Zecken. Eine weitere Maßnahme trägt diesem Anspruch Rechnung, nämlich dahingehend, dass zwischen zwei der Ecken der Karte ein kammartiger Bereich mit in der Ebene der Karte liegenden Zinken ausgebildet ist. Die Zinken sind in die Kante der Karte eingearbeitet und enden am Rand der Karte bzw. nahe des Randes der Karte, ohne dabei die Anordnung und Funktion der Aufnahmeschlitze zu beeinträchtigen.

In vorteilhafter Weise ist der kammartige Bereich wesentlich feingliedriger als der Aufnahmeschlitz ausgebildet. Dazu umfasst der kammartige Bereich Zinken mit äuβerst geringen Abständen bzw. Freiräumen dazwischen, wobei es denkbar ist, dass die Zinken mit parallelen Kanten zueinander verlaufen. In ganz besonders vorteilhafter Weise bilden die Zinken sich nach innen verjüngende Schlitze, so dass auch dem kammartigen Bereich bzw. den dortigen Zinken eine keilartige Wirkung zuzuschreiben ist. So lassen sich die Parasiten, insbesondere Larven oder kleinere Parasiten, mit dem kammartigen Bereich regelrecht von der Hautoberfläche oder aus dem Fell wegkämmen bzw. durch Hintergreifen aus der Haut herausziehen.

In Bezug auf eine konkrete Ausgestaltung des kammartigen Bereichs bzw. der Zinken ist es von weiterem Vorteil, wenn die Zinken zu den Ecken hin kürzer ausgebildet sind, d.h. sich kürzer in die Karte hinein erstrecken. Durch diese Maßnahme ist beidseitig gewährleistet, dass die angrenzenden Eckbereiche nicht unnötig im Material reduziert sind, so dass in Bezug auf die dortigen Aufnahmeschlitze eine hinreichende Stabilität bzw. Festigkeit der Karte gewährleistet ist.

In Bezug auf das Material der Karte ist es von ganz besonderem Vorteil, wenn diese aus elastischem Kunststoff gefertigt ist. Eine spritzgusstechnische Herstellung ist dabei zu bevorzugen.

Ebenso ist es denkbar, die Karte aus Holz oder aus Metall zu fertigen. Bei einer Ausführung aus Metall bietet sich Aluminium oder eine Aluminiumlegierung an. Die Karte sollte eine gewisse Elastizität, eine hinreichende Festigkeit sowie eine resistente Oberfläche aufweisen.

Bei der erfindungsgemäßen Vorrichtung handelt es sich um ein Werkzeug mit hoher Funktionalität in Bezug auf die zugrunde liegende Aufgabe. Darüber hinaus weist die Karte - beidseitig - eine hinreichend große Oberfläche auf. Diese Fläche lässt sich in idealer Weise als Informationsträger, insbesondere als Werbefläche bzw. Werbeträger, verwenden. Somit bietet es sich an, die Karte im Sinne eines Werbegeschenks oder grundsätzlich mit Werbung zu bedrucken, beispielsweise mit der Werbung eines Zoofachgeschäfts, eines Tierarztes, eines Futterherstellers, etc. Des Weiteren bietet es sich an, entsprechend der Form der Karte ein Etui vorzusehen, welches aus Karton, Kunststoff, Stoff, Leder, etc. gefertigt sein kann. Auch ist es denkbar, die Karte, möglichst in miniaturisierter Form, als Schlüsselanhänger auszuführen.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Draufsicht ein Ausführungsbeispiel einer er- findungsgemäßen Vorrichtung zum Entfernen von Zecken,
- Fig. 2: in einer quasi perspektivischen Ansicht den Gegenstand aus Fig. 1 von oben und
- Fig. 3: in einer quasi perspektivischen Ansicht den Gegenstand aus Fig. 1 von unten.

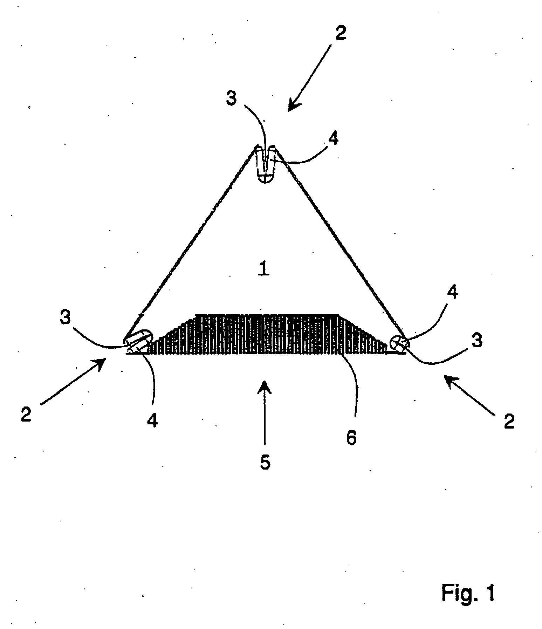

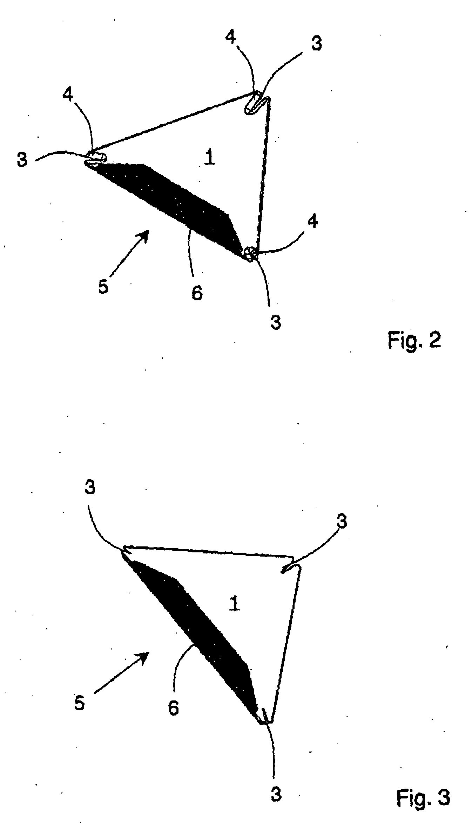

Die Figuren 1 bis 3 zeigen ein Ausführungsbeispiel einer erfindungsgemäßen Vorrichtung zum Entfernen von Zecken, wobei die Vorrichtung eine zur Handhabung dienende Karte 1 umfasst. Die Figuren zeigen deutlich, dass die Karte 1 dreieckig ausgeführt ist, wobei jede der Ecken 2 einen Aufnahmeschlitz 3 zum Hintergreifen der Zecke aufweist. Die insgesamt drei Aufnahmeschlitze 3 haben eine unterschiedliche Größe, um nämlich unterschiedlich große Zecken, d.h. Zecken in unterschiedlichen Stadien, sicher hintergreifen und herausziehen zu können.

Die Figuren zeigen des Weiteren deutlich, dass die die Aufnahmeschlitze 3 bildenden Kanten 4 von einer Seite her, d.h. von der Oberseite her, abgeschrägt bzw. angefast sind. Dadurch wird das Hintergreifen der Zecken begünstigt.

Die Figuren zeigen auch, dass zwischen zwei der Ecken 2 ein kammartiger Bereich 5 ausgebildet ist, der Zinken 6 umfasst, die in der Ebene der Karte 1 liegen. Die Zinken 6 enden am Rand der Karte 1 und haben beidseitig sich nach innen verjüngende Schlitze, so dass sie eine keilartige Wirkung haben. Außerdem zeigen die. Figuren deutlich, dass sich die Zinken 6 zu den Ecken 2 hin verjüngen bzw. kürzer ausgebildet sind, damit der die Aufnahmeschlitze 3 umfassende Bereich nicht unnötig geschwächt bzw. zu sehr im Material reduziert ist. Eine hinreichende Stabilität der angrenzenden Ecken 2 und der dortigen Aufnahmeschlitze 3 ist gewährleistet.

Des Weiteren sei angemerkt, dass die Karte 1 aus elastischem Kunststoff spritzgusstechnisch gefertigt ist. Die Karte 1 kann beliebige Aufdrucke bzw. Informationen, z.B. Werbung, tragen.

Hinsichtlich weiterer Merkmale, die sich den Figuren nicht entnehmen lassen, sei zur Vermeidung von Wiederholungen auf den allgemeinen Teil der Beschreibung verwiesen.

### Bezugszeichenliste

- 1: Karte
- 2: Eck(en) der Karte
- 3: Aufnahmeschlitz
- 4: Kanten des Aufnahmeschlitzes
- 5: kammartiger Bereich
- 6: Zinken

## Patentansprüche

1. Vorrichtung zum Entfernen von Parasiten, insbesondere von Zecken, vom Menschen oder vom Tier, mit einer zur Handhabung dienenden Karte (1) und mit mindestens einem in einem Eckbereich der Karte (1) ausgebildeten Aufnahmeschlitz (3) zum Hintergreifen oder Ergreifen des Parasiten, insbesondere der Zecke bzw. des Zeckenkörpers,
**dadurch gekennzeichnet, dass** die Karte (1) in Form eines gleichseitigen Dreiecks ausgeführt und der Aufnahmeschlitz (3) in einer der Ecken (2) ausgebildet ist.

2. Vorrichtung nach Anspruch 1, **dadurch gekennzeichnet, dass** die Karte (1) eine Dicke im Bereich von 1 bis 2 mm, vorzugsweise etwa 1,5 mm, hat.

3. Vorrichtung nach Anspruch 1 oder 2, **dadurch gekennzeichnet, dass** sich der Aufnahmeschlitz (3) von einer breiten Öffnung bis zu einem spitz oder leicht abgerundet zulaufenden Ende vorzugsweise konisch oder bogenförmig verjüngt.

4. Vorrichtung nach einem der Ansprüche 1 bis 3, **dadurch gekennzeichnet, dass** die den Aufnahmeschlitz (3) bildenden Kanten (4) vorzugsweise von einer Seite der Karte (1) her abgeschrägt bzw. angefast sind.

5. Vorrichtung nach einem der Ansprüche 1 bis 4, **dadurch gekennzeichnet, dass** der Aufnahmeschlitz (3) im Sinne einer Winkelhalbierenden in die jeweilige Ecke (2) eingearbeitet ist.

6. Vorrichtung nach einem der Ansprüche 1 bis 5, **dadurch gekennzeichnet, dass** die Karte (1) an zwei oder drei ihrer Ecken (2) Aufnahmeschlitze (3) in unterschiedlichen Größen, d.h. mit unterschiedlichen Öffnungen und Tiefen, aufweist.

7. Vorrichtung nach einem der Ansprüche 1 bis 6, **dadurch gekennzeichnet, dass** zwischen zwei der Ecken (2) ein kammartiger Bereich (5) mit in der Ebene der Karte (1) liegenden Zinken (6) ausgebildet ist,
wobei die Zinken (6) in die Kante der Karte (1) eingearbeitet sein können und somit am Rand der Kante (1) enden.

8. Vorrichtung nach Anspruch 7, **dadurch gekennzeichnet, dass** durch die Zinken (6) sich nach innen verjüngende Schlitze ausgebildet sind.

9. Vorrichtung nach Anspruch 7 oder 8, **dadurch gekennzeichnet, dass** die Zinken (6) zu den Ecken (2) hin kürzer ausgebildet sind, d.h. sich kürzer in die Karte (1) hinein erstrecken.

10. Vorrichtung nach einem der Ansprüche 1 bis 9, **dadurch gekennzeichnet, dass** die Karte (1) aus vorzugsweise elastischem Kunststoff gefertigt ist.

11. Vorrichtung nach einem der Ansprüche 1 bis 9, **dadurch gekennzeichnet, dass** die Karte (1) aus Holz gefertigt ist.

12. Vorrichtung nach einem der Ansprüche 1 bis 9, **dadurch gekennzeichnet, dass** die Karte (1) aus Metall, vorzugsweise aus Aluminium oder aus einer Aluminiumlegierung, gefertigt ist.

13. Vorrichtung nach einem der Ansprüche 1 bis 12, **dadurch gekennzeichnet, dass** zumindest eine Seite der Karte (1) als Informationsträger, insbesondere als Werbefläche, ausgeführt ist.

14. Vorrichtung nach einem der Ansprüche 1 bis 13, **gekennzeichnet durch** ein Etui zum Einstecken der Karte (1),
wobei das Etui aus Karton, Kunststoff, Stoff oder Leder gefertigt sein kann.

## Claims

1. Device for removing parasites, in particular ticks, from a human or from an animal, having a board (1) which is used for handling and having at least one receiving slot (3) which is formed in a corner region of the board (1) for engaging behind or grasping the parasite, in particular the tick or the tick body,
**characterised in that** the board (1) is in the form of an equilateral triangle and the receiving slot (3) is formed in one of the corners (2).

2. Device according to claim 1, **characterised in that** the board (1) has a thickness in the range from 1 to 2 mm, preferably of approximately 1.5 mm.

3. Device according to claim 1 or claim 2, **characterised in that** the receiving slot (3) tapers from a wide opening to a pointed or slightly rounded end, preferably in a conical or curved manner.

4. Device according to any ono of claims 1 to 3,
**characterised in that** the edges (4) which form the receiving slot (3) are preferably chamfered or bevelled from one side of the board (1).

5. Device according to any one of claims 1 to 4,
**characterised in that** the receiving slot (3) is formed in the respective corner (2) in the manner of an angle bisector.

6. Device according to any one of claims 1 to 5,
**characterised in that** the board (1) has at two or three of the corners (2) thereof receiving slots (3) of different sizes, that is to say, with different openings and depths.

7. Device according to any one of claims 1 to 6,
**characterised in that**, between two of the corners (2), there is formed a comb-like region (5) having teeth (6) which are located in the plane of the board (1),
the teeth (6) being able to be formed in the edge of the board (1) and consequently terminating at the edge of the board (1).

8. Device according to claim 7, **characterised in that** slots which taper inwards are formed by the teeth (6).

9. Device according to claim 7 or claim 8, **characterised in that** the teeth (6) are constructed so as to be shorter towards the corners (2), that is to say, extend over a shorter distance into the board (1).

10. Device according to any one of claims 1 to 9,
**characterised in that** the board (1) is produced from preferably resilient plastics material.

11. Device according to any one of claims 1 to 9,
**characterised in that** the board (1) is produced from wood.

12. Device according to any one of claims 1 to 9,
**characterised in that** the board (1) is produced from metal, preferably from aluminium or from an aluminium alloy.

13. Device according to any one of claims 1 to 12, **characterised in that** at least one side of the board (1) is constructed as an information carrier, in particular as an advertising surface.

14. Device according to any one of claims 1 to 13, **characterised by** a case for insertion of the board (1), the case being able to be produced from cardboard, plastics material, fabric or leather.

## Revendications

1. Dispositif pour enlever des parasites, en particulier des tiques, de l'homme ou de l'animal, comprenant une carte (1) servant à la manipulation et au moins une fente de logement (3) conçue dans une zone d'angle de la carte (1) pour enserrer de l'arrière ou saisir le parasite, en particulier la tique ou le corps de la tique,
**caractérisé en ce que** la carte (1) est réalisée sous la forme d'un triangle équilatéral et la fente de logement (3) est conformée dans l'un des coins (2).

2. Dispositif selon la revendication 1, **caractérisé en ce que** la carte (1) a une épaisseur de l'ordre de 1 à 2 mm, de préférence d'environ 1,5 mm.

3. Dispositif selon la revendication 1 ou 2, **caractérisé en ce que** la fente de logement (3) se rétrécit de préférence en forme de cône ou en forme d'arc depuis une large ouverture jusqu'à une extrémité se terminant de façon pointue ou légèrement arrondie.

4. Dispositif selon l'une quelconque des revendications 1 à 3, **caractérisé en ce que** les arêtes (4) formant la fente de logement (3) sont biseautées ou chanfreinées de préférence à partir d'un côté de la carte (1).

5. Dispositif selon l'une quelconque des revendications 1 à 4, **caractérisé en ce que** la fente de logement (3) est intégrée, dans le sens d'une bissectrice, dans le coin (2) respectif.

6. Dispositif selon l'une quelconque des revendications 1 à 5, **caractérisé en ce que** la carte (1) présente sur deux ou trois de ses coins (2) des fentes de logement (3) dans des grandeurs différentes, c'est-à-dire avec des ouvertures et des profondeurs différentes.

7. Dispositif selon l'une quelconque des revendications 1 à 6, **caractérisé en ce qu'**une zone (5) en forme de peigne est conformée avec des dents (6) situées dans le plan de la carte (1) entre deux des coins (2),
les dents (6) pouvant être intégrées dans l'arête de la carte (1) et se terminant ainsi sur le bord de l'arête (1).

8. Dispositif selon la revendication 7, **caractérisé en ce que** des fentes se rétrécissant vers l'intérieur sont conformées par les dents (6).

9. Dispositif selon la revendication 7 ou 8, **caractérisé en ce que** les dents (6) sont conformées plus courtes en direction des coins (2), c'est-à-dire s'étendent de façon plus courte à l'intérieur de la carte (1).

10. Dispositif selon l'une quelconque des revendications 1 à 9, **caractérisé en ce que** la carte (1) est fabriquée à base de matière synthétique de préférence élastique.

11. Dispositif selon l'une quelconque des revendications 1 à 9, **caractérisé en ce que** la carte (1) est fabriquée en bois.

12. Dispositif selon l'une quelconque des revendications 1 à 9, **caractérisé en ce que** la carte (1) est fabriquée en métal, de préférence en aluminium ou à base d'un alliage d'aluminium.

13. Dispositif selon l'une quelconque des revendications 1 à 12, **caractérisé en ce qu'**au moins un côté de la carte (1) est conçu sous forme de support d'information, en particulier sous forme de surface publicitaire.

14. Dispositif selon l'une quelconque des revendications 1 à 13, **caractérisé par** un étui pour l'enfichage de la carte (1),
l'étui pouvant être fabriqué en carton, matière synthétique, tissu ou cuir.
